(19) Europäisches Patentamt
European Patent Office
Office européen des brevets

(11) Publication number: **0 112 685**
**B1**

(12) **EUROPEAN PATENT SPECIFICATION**

(45) Date of publication of patent specification: **23.04.86**

(21) Application number: **83307600.3**

(22) Date of filing: **14.12.83**

(51) Int. Cl.[4]: **C 07 D 498/04,** A 61 K 31/42 // (C07D498/04, 263:00, 205:00),(A61K31/42, 31:43),(A61K31/42, 31:545)

(54) **Compounds containing beta-lactams.**

(30) Priority: **24.12.82 GB 8236794**
**27.01.83 GB 8302303**

(43) Date of publication of application:
**04.07.84 Bulletin 84/27**

(45) Publication of the grant of the patent:
**23.04.86 Bulletin 86/17**

(84) Designated Contracting States:
**BE CH DE FR GB IT LI NL SE**

(56) References cited:
**EP-A-0 002 370**
**GB-A-1 572 289**

(73) Proprietor: **BEECHAM GROUP PLC**
**Beecham House Great West Road**
**Brentford Middlesex TW8 9BD (GB)**

(72) Inventor: **Davies, John Sydney**
**17 Beaufort Road**
**Reigate Surrey (GB)**
Inventor: **Hunter, Pamela Ann**
**Caniper Cottage Mill Lane**
**Lower Beeding Horsham Sussex (GB)**

(74) Representative: **Hesketh, Alan, Dr. et al**
**European Patent Attorney Beecham Pharmaceuticals Great Burgh Yew Tree Bottom Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 112 685 B1

Courier Press, Leamington Spa, England.

**Description**

This invention relates to a class of novel β-lactam compounds, to the process for their preparation and to pharmaceutical compositions containing them.

U.K. Patent Nos. 1,508,977 and 1,508,978 disclose *inter alia* clavulanic acid and its salts and esters. Clavulanic acid has the formula (A):

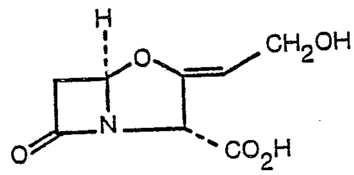

(A)

Ethers of clavulanic acid, its salts and esters are disclosed in U.K. patent specification No. 1 565 209 which are of formula (B):

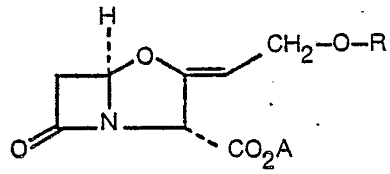

(B)

wherein R is an inert organic group of up to 18 carbon atoms and A is a group such that $CO_2A$ represents a carboxylic acid group or a salt or ester thereof.

The compounds of both formula (A) and formula (B) possess anti-bacterial and β-lactamase inhibitory activity.

We have now found a novel class of halogen containing compounds which not only have anti-bacterial and β-lactamase inhibitory activity but also have the advantage of exhibiting high and prolonged blood levels upon administration.

Accordingly, the present invention provides a compound of formula (I) or a salt or ester thereof:

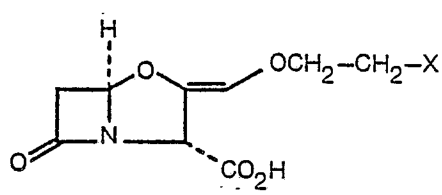

(I)

wherein X represents chlorine, bromine, or iodine. Preferably X is chlorine.

The major utility of the compound of formula (I) is as a pharmaceutical and, accordingly, the salts and esters of the compound of formula (I) are preferably pharmaceutically acceptable. The compound of formula (I) may also be used as an anti-bacterial or β-lactamase inhibitor in non-pharmaceutical uses such as, for example, as a disinfectant or paint additive; those salts and esters which are not normally considered to be pharmaceutically acceptable are suitable for this application.

Suitable pharmaceutically acceptable salts of the compounds of formula (I) include metal salts, eg aluminium, alkali metal salts such as sodium or potassium, alkaline earth metal salts such as calcium or magnesium and ammonium or substituted ammonium salts, for example those with lower alkylamines such as triethylamine, cycloalkylamines such as bicyclohexylamine, or with dibenzylamine, N,N-dibenzyl-ethylenediamine, l-ephenamine, N-ethylpiperidine, or N-benzyl-β-phenethylamine.

Particularly suitable pharmaceutically acceptable salts of the compound of formula (I) are the alkali metal salts such as lithium, sodium and potassium. The preferred pharmaceutically acceptable salts are the sodium and potassium salts.

Suitable pharmaceutically acceptable esters of the compounds of formula (I) include *in vivo* hydrolysable esters. Further suitable pharmaceutically acceptable esters include alkyl esters, such as for example $C_{1-6}$ alkyl esters.

Examples of suitable pharmaceutically acceptable in vivo hydrolysable ester groups include those which break down readily in the human body to leave the parent acid or its salt. Suitable ester groups of this type include those of part formula (i), (ii) and (iii):

$$\begin{array}{c} R^a \\ | \\ -CO_2CH-O.CO.R^b \end{array} \qquad (i)$$

$$\begin{array}{c} \quad\quad R^d \\ \quad\quad | \\ -CO_2R^c-N \\ \quad\quad | \\ \quad\quad R^e \end{array} \qquad (ii)$$

$$-CO_2CH_2OR^f \qquad (iii)$$

wherein $R^a$ is hydrogen, methyl, or phenyl, $R^b$ is $C_{1-6}$ alkyl, $C_{1-6}$ alkoxy or phenyl; or $R^a$ and $R^b$ together form a 1,2-phenylene group optionally substituted by one or two methoxy groups; $R^c$ represents $C_{1-6}$ alkylene optionally substituted with a methyl or ethyl group — $R^d$ and $R^e$ independently represent $C_{1-6}$ alkyl; $R^1$ represents $C_{1-6}$ alkyl. Examples of suitable in vivo hydrolysable ester group include for example acyloxyalkyl groups such as acetoxymethyl, pivaloyloxymethyl, α-acetoxyethyl and α-pivaloyloxyethyl groups; alkoxycarbonyloxyalkyl groups, such as ethoxycarbonyl-oxymethyl and α-ethoxycarbonyloxyethyl; dialkylamino-alkyl especially di-loweralkylamino alkyl groups such as dimethylaminoethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

The present invention also provides a pharmaceutical composition which comprises a compound of this invention and a pharmaceutically acceptable carrier.

The compositions of the invention include those in a form adapted for oral, topical or parenteral use and may be used for the treatment of the infection in animals especially mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accorance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as, a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam antibiotics for inclusion in such synergistic compositions include not only those known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, tricarcillin, cyclacillin, pirbenicillin, azlocillin, mezlocillin, sulbenicillin, piperacillin, and other well known penicillins including pro-drugs thereof such as their in vivo hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin,) or α-esters of carbenicillin, or ticarcillin, such as their phenyl or indanyl α-esters.

Suitable cephalosporins for inclusion in the compositions of this invention include, for example, cefatrizine, cephaloridine, cephalothin, cefazolin, cephalexin, cephacetrile, cephapirin, cephamandole

nafate, cephradine, 4-hydroxycephalexin, cephaloglycin, cefoperazone, and known cephalosporins or pro-drugs thereof.

Such compounds are frequently used in the form of a salt or hydrate or the like.

Naturally if the penicillin or cephalosporin present in the composition is not suitable for oral administration then the composition will be adapted for parenteral administration.

Highly favoured penicillins for use in the compositions of this invention include ampicillin, amoxycillin, carbenicillin, and ticarcillin,. Such penicillins may be used as a pharmaceutically aceptable salt such as the sodium salt. Alternatively the ampicillin, or amoxycillin, may be used in the form of fine particles of the zwitterionic form (generally as ampicillin, trihydrate or amoxycillin, trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt, for example the sodium salt.

When present together with a cephalosporin or penicillin, the ratio of a compound of the invention to the penicillin or cephalosporin agent may vary over a wide range of ratios, such as from 10:1 to 1:10, for example about 3:1, 2:1, 1:1, 1:2, 1:3, 1:4, 1:5 or 1:6 (wt/wt, based on pure free antibiotic equivalent). Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example, about 62.5, 100, 125, 150, 200 or 250 mg.

Compositions of this invention may be used for the treatment of infections of inter alia, the respiratory tract, the urinary tract and soft tissues in humans and mastitis in cattle.

Normally for adult human treatment between 50 and 3000 mg of the compounds of the invention will be administered each day of treatment but more usually between 100 and 1000 mg of the compounds of the invention will be administered per day, for example at 1—6 doses, more usually as 2, 3 or 4 doses. However for the treatment of more severe systemic infections or infections of particularly intransigent organisms higher doses may be used in accordance with clinical practice.

The penicillins or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug thereof and from 25 to 500 mg of a compound of this invention.

Most suitably this form of composition will contain ampicillin trihydrate, ampicillin anhydrate, sodium ampicillin, hetacillin, pivampicillin, hydrochloride, bacampicillin hydrochloride or talampicillin hydrochloride. Most suitable this form of the composition will contain a compound of the formula (I) when in crystalline form.

Most suitably the preceding composition will contain from 200 to 700 mg of the penicillin component. Most suitably the preceding composition will comprise from 50 to 250 mg of a compound of the formula (I) preferably in crystalline form.

Such compositions may be adapted for oral or parenteral use exccept when containing an in vivo hydrolysable ester of ampicillin or amoxycillin in which case the compositions will not be adapted for parenteral administration.

Another particularly favoured compositions of this invention will contain from 200 to 2000 mg of carbenicillin, ticarcillin or a pro-drug thereof and from 50 to 500 mg of a compound of this invention.

Suitably this form of composition will contain di-sodium carbenicillin. Suitably this form of the composition will contain di-sodium ticarcillin.

More suitably this form of the composition will contain from 75 to 250 mg of a compound of the formula (I) preferably in crystalline form. Such compositions containing di-salts of carbenicillin and ticarcillin will be adapted for parenteral administration.

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compound of this invention is Staphylococcus aureus. The other organisms named are more readily treated by using a synergistically effective amount of the compound of this invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of formula (I) or a salt or ester thereof, which process comprises halogenation (i.e. chlorination, bromination or iodination) of a compound of formula (II):

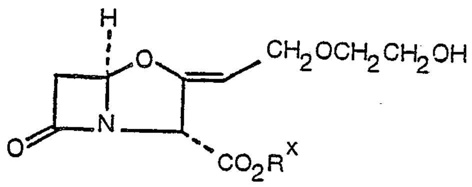

(II)

wherein $R^x$ is a carboxy protecting group;

and thereafter where necessary carrying out one or more of the following steps:

(a) removing the carboxy protecting group $R^x$;

(b) converting a salt to the free craboxylic acid or to an ester, or to a different salt.

The halogenation may be achieved by using halogenating reagents such as thionyl halide, e.g. thionyl chloride, oxyhalides such as phosphorous oxychloride, oxalylhalide, for example oxalylchloride. Chlorination may also be achieved by reaction of a compound of formula (II) with methane sulphonyl chloride or toluene sulphonyl chloride in dimethyl formamide. The chlorination is suitably carried out in the presence of an organic base such as pyridine.

The preceding reaction normally takes place in hexamethylphosphoramide. The reaction is generally carried out at a ambient or elevated temperature for example +20°C to +80°C, and preferably at an elevated temperature, for example +30°C to +50°C.

The compound of formula (II) may itself be prepared by a method described in European patent publication No. 0002370.

Suitable carboxy protecting groups for the group $—CO_2R^x$ in formula (II) include ester derivatives of the carboxylic acid. The derivative is preferably one which may readily be cleaved at a later stage of the reaction.

Suitable ester-forming carboxylic-blocking groups are those which may be removed under conventional conditions. Such groups for $R^x$ include benzyl, p-methoxybenzyl, 2,4,6-trimethylbenzyl, 3,5-dit-butylbenzyl, 4-pyridylmethyl, allyl, diphenylmethyl, triphenymethyl, 2-benzyloxphenyl, 4-methylthiophenyl, methoxymethyl, a silyl, or phosphorus-V-containing group, or methyl or ethyl or an *in vivo* hydrolysable ester as hereinbefore defined.

The carboxylic group or a salt thereof may be regenerated from any of the above esters by usual methods appropriate to the particular $R^x$ group, for example, base-catalysed hydrolysis, or by enzymically-catalysed hydrolysis, or by hydrogenation.

Acids within formula (I) may also be prepared by the careful acidification of a corresponding salt such as the sodium salt.

Salts within formula (I) may be prepared by treatment of an acid within formula (I) with, for example, sodium ethyl hexanoate or potassium ethyl hexanoate. Salts within formula (I) may also be prepared by salt exchange in conventional manner, for example a solution of the lithium salt in water may be passed through a bed or ion exchange resin in the sodium form (e.g. Amberlite*) 120; a sodium salt of a sulphonate polystyrene divinyl benzene co-polymer) in about ten-fold excess until elution is complete; the resulting sodium salt may be obtained by freeze drying or by evaporation. Similarly, a sodium salt may be converted to a lithium salt or to a potassium salt in similar manner.

(* Amberlite is a trade name.)

The following Examples illustrate the invention. In the Examples temperatures are given in °C.

### Preparation 1

p-Nitrobenzyl-9-0-(2-hydroxyethyl)clavulanate

A solution of p-nitrobenzyl clavulanate (30g) and ethylene oxide (6.7g) in dichloromethane (200 ml) was cooled to 0°C and treated with boron trifluorideetherate (1.8ml). The mixture was stirred at 0° for ½ hr. and then at room temperature for ¼ hr. The mixture was then shaken with excess aqueous sodium bicarbonate solution and the organic phase separated. The aqueous phase was thoroughly extracted with chloroform and the combined organic phases washed with brine, dried over anhydrous magnesium sulphate and evaporated. Chromatography on silica gel using ethyl acetate-petrol 1:1 gave the title compound as a crystalline compound (5.08 g; 15%) m.p. 69—70° (ethylacetate-petrol); $[\alpha]^{20}$ + 40.6° (C. 1.0, $CHCL_3$); $\nu_{max}$ ($CHCl_3$) 3450, 1805, 1760, 1695 and 1605cm$^{-1}$; δ ($CDCl_3$) 2.07 (1H, broads, —OH), 3.06 (1H, d, $J$=17Hz, 6β-C<u>H</u>), 3.35—3.85 (5H, m), 4.12 (2H, d, $J$=7Hz, 9-C$\underline{H_2}$), 4.88 (1H, t, J=7Hz, 8-C<u>H</u>), 5.16 (1H, s, 3-C<u>H</u>), 5.32 (2H, s, Ar—C$\underline{H_2}$), 5.71 (1H, d, J=3Hz, 5-CH), 7.55 and 8.29 (4H, 2d, AR—<u>H</u>).

### Example 1

p-Nitrobenzyl-9-0-(2-chloroethyl)clavulanate

p-Nitrobenzyl-9-0-(2-hydroxyethyl)clavulanate (0.83g) was dissolved in dimethylformamide (20ml) and the solution warmed to 60°. At this temperature methanesulphonyl chloride (0.5g) and pyridine (0.35g) were added and the mixture stirred at 60° for ½ hr. The solution was allowed to cool and diluted with excess ethyl acetate. The organic solution was washed successively with water, dilute hydrochloric acid, sat. aqueous sodium bicarbonate and finally brine. After drying over anhydrous magnesium sulphate the organic solution was evaporated to give an oil. Chromatography on silica gel using ethyl acetate-petrol 1:1

as eluant gave the title compound as a colourless oil (0.161g; 18%), $[\alpha]^{20}$ + 36.5 (C.1.0, $CHCl_3$); $\nu_{max}$ ($CHCl_3$) 1805, 1755, 1695 and 1605 $cm^{-1}$; $\delta$($CDCl_3$), 3.06 (1H, d, $J$=17Hz, 6-β-C<u>H</u>), 3.50—3.62 (5H, m), 4.13 (2H, d, $J$=7Hz, 9-C<u>H</u>$_2$), 4.84 (1H, t, $J$=7Hz, 8-C<u>H</u>), 5.12 (1H, s, 3-C<u>H</u>), 5.28 (2H, s, Ar—$CH_2$), 5.68 (1H, d, J=3Hz, 5-C<u>H</u>), 7.5 and 8.23 (4H, 2d, Ar—<u>H</u>).

Example 2

Lithium-9-0-[2-chloroethyl]clavulanate

p-Nitrobenzyl-9-0-(2-chloroethyl)clavulanate (90mg) was dissolved in tetrahydrofuran (10 ml) with stirring, ice-cooled and treated with 1M aqueous ammonium chloride solution (3 ml) and iron powder (0.37g). After 20 minutes a further amount of 1M ammonium chloride solution (1.2ml) and iron powder (0.3g) were added and the stirring continued for 45 minutes. Ethyl acetate (100ml) was added and $H_2S$ bubbled through the mixture for 10 minutes with ice-cooling. The midture was filtered through Celite and the residues washed with water (20 ml). The aqueous layer of the filtrate (including washings) was saturated with NaCl, acidified with 1N hydrochloric acid to pH 2.5 and separated from the organic layer. The aqueous layer was further extracted with ethyl acetate (2 × 30ml) and the combined ethyl acetate extracts dried over magnesium sulphate. After filtration the ethyl acetate was extracted with pH7 phosphate buffer. The combined aqueous extracts were saturated with NaCl acidified to pH 2.5 with 1*N* hydrochloric acid and extracted with ethyl acetate. The ethyl acetate extracts were dried over $MgSO_4$ and evaporated and the residue quickly taken up in tetrahydrofuran (10 ml) and water (5 ml). This solution was brought to pH7 by addition of lithium hydroxide solution, washed with ether and freeze dried to give the title compound (40 mg; 63%). Identical in all respects (ir, nmr, etc.) to the compound described in example 4.

Example 3

Benzyl-9-0-[2-chloroethyl]clavulanate

Benzyl-9-0-(2-hydroxyethyl)clavulanate (2.12g) in dimethylformamide was treated at 60° with methanesulphonyl chloride (0.76g) and then pyridine (0.52g). Stirring was continued at this temperature for ½ hr. and then the solution was poured into an excess of ethyl acetate. The organic solution was washed successively with water, 3*N* hydrochloric acid, saturated aqueous sodium bicarbonate and finally brine. After drying over anhydrous magnesium sulphate the solution was evaporated to give an oil. Chromatography on silica gel using ethyl acetate-petrol 1:1 gave the title compound as an oil (0.28g; 13%), $[\alpha]^{20}$ + 46.2° [C. 1.0, $CHCl_3$], $\nu_{max}$ ($CHCl_3$) 1800, 1745 and 1695 $cm^{-1}$; $\delta$ ($CDCl_3$), 3.03 (1H, d, $J$=17Hz, 6β-C<u>H</u>), 3.5—3.61 (5H, m), 4.12 (2H, d, J=7Hz, 9-C<u>H</u>$_2$), 4.81 (1H, t, $J$=7Hz, 8-C<u>H</u>), 5.10 (1H, s, 3-C<u>H</u>), 5.18 (2H, s, Ar—<u>C</u>H$_2$), 5.68 (1H, d, $J$=3Hz, 5-C<u>H</u>), 7.35 (5H, s, Ar—<u>H</u>).

The same experiment was conducted using the following ingredients: Benzyl-9-0-2-(2-hydroxyethyl) clavulanate (1.9g; 0.0057m), DMF (30ml), methanesulphonyl chloride (1.3g; 0.0114m), pyridine (0.9g; 0.0114m). Benzyl-9-0-2-(2-chloroethyl)-clavulanate (0.570g; 29%) was isolated.

Example 4

Lithium 9-0-(2-chloroethyl)clavulanate

Benzyl-9-0-[2-chloroethyl]clavulanate (0.212g) in tetrahydrofuran (10ml) was hydrogenated over 10% palladium-charcoal (0.106g) for ½ hr. The solution was filtered through celite and the filter-cake washed with tetrahydrofuran. The combined filtrates were diluted with water and a solution of aqueous lithium hydroxide added until pH 7 was attained. The tetrahydrofuran was evaporated and the aqueous solution washed with ether and freeze-dried to give the title compound as a pale beige solid (0.114g; 68%); $\nu_{max}$ (KBr) 1765, 1690 and 1615$cm^{-1}$; $\delta$ ($D_2O$), 3.09 (1H, d, $J$=17Hz, 6β-C<u>H</u>), 3.44—3.72 (5H, m), 4.17 (2H, d, $J$=7Hz 9-C<u>H</u>$_2$), 4.89 (1H, t, $J$=7Hz, 8-C<u>H</u>), 4.96 (1H, s, 3-C<u>H</u>), 5.71 (1H, d, $J$=3Hz, 5-C<u>H</u>).

Example 5

p-Nitropbenzyl-9-0-(2-Chloroethyl) clavulanate

p-Nitrobenzyl-9-0-(2-hydroxyethyl) clavulanate (1g; 0.0026 mol) in dry hexamethylphosphoramide (HMPA) (15ml) was treated, undernitrogen, with thionyl chloride (0.256ml; 1.2 equivalents) and pyridine (0.18ml; 1.2 equivalents). The mixture was stirred at room temperature for 4 hours, poured onto saturated sodium bicarbonate cooled with ice, and extracted with ethyl acetate (2 × 50ml). The organic layer was washed with dilute hydrochloric acid, water and brine, dried over anhydrous magnesium sulphate and evaporated to give an orange oil. Chromatography of the residue on silica-gel using ethyl acetate-petrol (1:1) as eluant gave the title ester as a colourless oil (0.69g; 67%).

Biological Data

The compound of Example 4 was administered either orally (by gastric lavage) or subcutaneously as a solution to groups of mice. At intervals after administration, 5 mice which had received the compound subcutaneously and 10 mice which had received the compound orally were sacrificed, and blood collected from the axillary region. The blood was assayed for content of compound 4 using a biological β-lactamase inhibition-zone assay technique. The mean values (± standard deviation — SD) are tabluated below.

When administered by either an oral or parenteral route, the compound of Example 4 produced high and prolonged levels in the blood of mice.

TABLE 1

Mean blood levels in mice (µg/ml) following oral administration of the compound of Example 4 of the present invention at a dosage rate of 20 mg/kg.

| | Time after administration (mins) | | | | | | |
|---|---|---|---|---|---|---|---|
| | 15 | 30 | 45 | 60 | 90 | 120 | AUC |
| average for 10 mice | 16.57 | 10.46 | 7.94 | 7.42 | 3.91 | 3.2 | 14.28 |
| ± S.D. | 6.8 | 2.39 | 2.83 | 2.82 | 1.14 | 1.27 | |

TABLE 2

Mean blood levels in mice (µg/ml) following subcutaneous administration of the compound of Example 4 of the present invention at a dosage rate of 10 mg/kg.

| | Time after administration (mins) | | | | | | | |
|---|---|---|---|---|---|---|---|---|
| | 10 | 20 | 30 | 45 | 60 | 90 | 120 | AUC |
| average for 5 mice | 22.8 | 17.3 | 13.3 | 7.7 | 5.2 | 2.7 | 1.8 | 15.1 |
| ± S.D. | 3.98 | 3.6 | 1.98 | 0.54 | 1.08 | 0.82 | 1.14 | |

**Claims**

1. A compound of formula (I) or a salt or ester thereof:

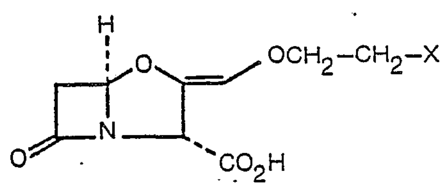

(I)

wherein X represents chlorine, bromine, or iodine.

2. A compound of formula (I) or a pharmaceutically acceptable salt or pharmaceutically acceptable ester thereof.

3. A compound as claimed in claim 1 or claim 2 wherein X is chlorine.

4. The sodium or potassium salt of a compound of formula (I).

5. The lithium salt of a compound of formula (I).

6. A pharmaceutical composition which comprises a compound as claimed in claim 2 and a pharmaceutically acceptable carrier.

7. A pharmaceutical composition as claimed in claim 6 which further comprises a penicillin or cephalosporin.

8. A process for the preparation of a compound as claimed in claim 1 which process comprises halogenation of a compound of formula (II):

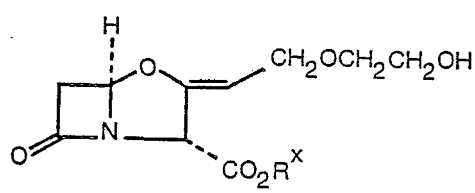

(II)

and thereafter where necessary carrying out one or more of the following steps:

(a) removing the carboxy protecting group $R^x$

(b) converting a salt to the free carboxylic acid or to an ester, or to a different salt.

9. A compound as claimed in claim 2 for use in treating bacterial infections.

10. The non-pharmaceutical anti-bacterial use of a compound as claimed in claim 1.

**Patentansprüche**

1. Eine Verbindung der Formel (I) oder deren Salz oder Ester

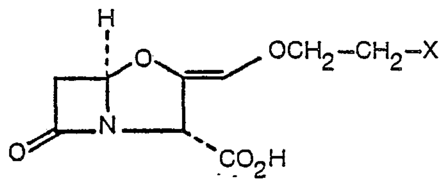

(I)

in der X Chlor, Brom oder Jod bedeutet.

2. Eine Verbindung der Formel (I) oder deren pharmazeutisch verträgliches Salz oder deren pharmazeutisch verträglicher Ester.

3. Eine Verbindung wie in Anspruch 1 oder Anspruch 2 beansprucht, wobei X Chlor ist.

4. Das Natrium- oder Kaliumsalz einer Verbindung der Formel (I).

5. Das Lithiumsalz einer Verbindung der Formel (I).

6. Eine pharmazeutische Zusammensetzung, die eine Verbindung wie in Anspruch 2 beansprucht und ein pharmazeutisch verträgliches Trägermaterial umfaßt.

7. Eine pharmazeutische Zusammensetzung wie in Anspruch 6 beansprucht, die weiterhin ein Penicillin oder Cephalosporin umfaßt.

8. Eine Verfahren zur Herstellung einer Verbindung wie in Anspruch 1 beansprucht, wobei das Verfahren die Maßnahme einer Halogenierung einer Verbindung der Formel (II)

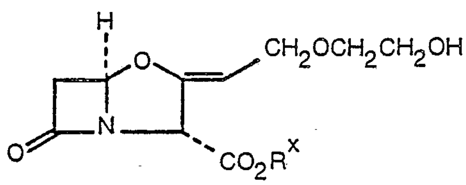

(II)

und danach gegebenenfalls einer Durchführung einer oder mehrerer der folgenden Stufen umfaßt:

(a) Entfernen der Carboxy-Schutzgruppe $R^x$

(b) Umwandeln eines Salzes in die freie Carbonsäure oder in einen Ester oder in ein anderes Salz.

9. Eine Verbindung wie in Anspruch 2 beansprucht zur Verwendung bei einer Behandlung bakterieller Infektionen.

10. Die nicht-pharmazeutische antibakterielle Verwendung einer Verbindung wie in Anspruch 1 beansprucht.

**Revendications**

1. Composé de formule (I) ou un sel ou un ester de celui-ci, représenté par la formule ci-après:

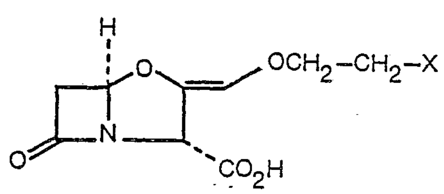

(I)

dans laquelle X est un atome de chlore, de brome ou d'iode.

2. Composé de formule (I) ou un sel acceptable du point de vue pharmaceutique ou un ester acceptable du point de vue pharmaceutique de celui-ci.

3. Composé suivant la revendication 1 ou 2, caractérisé en ce que X est un atome de chlore.

4. Sel de sodium ou de potassium d'un composé de formule (I).

5. Sel de lithium d'un composé de formule (I).

6. Composition pharmaceutique, caractérisée en ce qu'elle comprend un composé suivant la revendication 2 et un support acceptable du point de vue pharmaceutique.

7. Composition pharmaceutique suivant la revendication 6, caractérisée en ce qu'elle comprend de plus une pénicilline ou une céphalosporine.

8. Procédé de préparation d'un composé suivant la revendication 1, caractérisé en ce qu'il comprend l'halogénation d'un composé de formule (II)

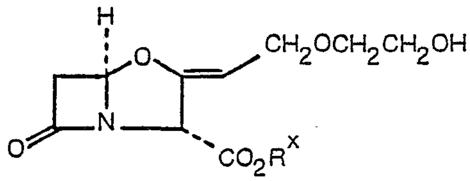

(II)

et éventuellement après, si cela est nécessaire, la réalisation d'une ou plusieurs des étapes suivantes:

(a) élimination du groupe $R^x$ protégeant la fonction carboxy

(b) conversion d'un sel en l'acide carboxylique libre ou en un ester ou en un sel différent.

9. Composé suivant la revendication 2, utile pour traiter des infections bactériennes.

10. Utilisation anti-bactérienne non-pharmaceutique d'un composé suivant la revendication 1.